# EUROPÄISCHE PATENTANMELDUNG

(11) **EP 4 124 333 A1**
(43) Veröffentlichungstag der Anmeldung: **01.02.2023**
(21) Anmeldenummer: 22174392.5
(22) Anmeldetag: 19.05.2022
(51) Int. Cl.: A61K 8/04, A61K 8/73, A61K 8/92, A61K 8/60, A61Q 5/06

(54) **HAARSCHAUMFESTIGER ENTHALTEND EIN NATÜRLICHES STYLING-POLYMER**

(30) Priorität: 23.06.2021 DE 102021206450
(71) Anmelder: Beiersdorf AG, 20245 Hamburg (DE)
(72) Erfinder: Budnick, Nina, Hamburg 22147 (DE); Behrendt, Pia, Hamburg 22455 (DE); Nemnich, Julia, 22589 Hamburg (DE)
(74) Vertreter: Beiersdorf AG

(57) **Zusammenfassung**

Haarschaumfestiger enthaltend wenigstens ein natürliches Styling-Polymer. Die Haarschaumfestiger zeichnen sich durch gute Festigungs- und Halteeigenschaften aus.

## Beschreibung

Die vorliegende Erfindung beschreibt eine kosmetische Zusammensetzung in Form eines Haarschaumfestigers zum Stylen von menschlichen Haaren, die ein natürliches Styling-Polymer enthält. Der Haarschaumfestiger zeichnet sich durch gute Festigungs- und Halteeigenschaften aus.

Haare sind ein wichtiger Teil des menschlichen Körpers. Der ganze menschliche Körper mit Ausnahme der Lippen, der Handinnenflächen und der Fußsohlen, ist behaart, zum Großteil allerdings mit kaum sichtbaren Wollhärchen. Wegen der vielen Nervenenden an der Haarwurzel reagieren Haare empfindlich auf äußere Einflüsse wie Wind oder Berührung und sind daher ein nicht zu unterschätzender Bestandteil des Tastsinns. Die wichtigste Funktion des menschlichen Kopfhaares dürfte allerdings heute darin bestehen, das Aussehen des Menschen in charakteristischer Weise mitzugestalten. Ähnlich wie die Haut erfüllt es eine soziale Funktion, da es über sein Erscheinungsbild erheblich zum Selbstwertgefühl des Individuums beiträgt.

Das Haar besteht aus dem frei aus der Haut herausragenden Haarschaft - dem keratinisierten (toten) Teil, der das eigentlich sichtbare Haar darstellt - und der in der Haut steckenden Haarwurzel - dem lebenden Teil, in dem das sichtbare Haar ständig neu gebildet wird. Der Haarschaft ist aus drei Schichten aufgebaut: Der zentrale Teil wird als Haarmark oder Medulla bezeichnet. Dieser Teil ist allerdings beim Menschen zurückgebildet und fehlt oft. An den zentralen Teil schließt sich die Haarrinde oder Cortex an. Diese Faserschicht besteht aus verhornten Faserzellen und bestimmt die Festigkeit und Elastizität des Haares. In dieser Schicht sind auch die Farbpigmente enthalten. Außen um die Faserschicht ist die Schuppenschicht oder Cuticula angeordnet. Diese Schicht ist mehrlagig, sehr dünn und durchsichtig.

Das menschliche Haar ist, sofern keine krankhaften Veränderungen vorliegen, in seinem frisch nachgewachsenen Zustand praktisch nicht zu verbessern. Der in der Nähe der Kopfhaut befindliche Teil eines Haares weist dementsprechend eine nahezu geschlossene Schuppenschicht auf.

Natürliches Haar hat in vielen Fällen wenig eigenes Volumen und bei langem Haar hängt es oftmals recht schlaff und gerade vom Kopf herab. Viele Menschen wünschen sich aber Haare, die mehr Volumen aufweisen und sich auf gefällige Art formen (stylen) lassen. Dies kann grundsätzlich auf zweierlei Weise erzielt werden, zum einen gibt es permanente Haarverformungsverfahren, beispielsweise die Dauerwelle und nicht permanente Verfahren, die die Frisur des Haares nur für eine begrenzte Zeit formen und fixieren. Um eine nicht-permanente Frisurgestaltung zu erreichen, werden mehrere Produktformen zur Verfügung gestellt, die einzeln oder in Kombination miteinander verwendet werden können. Zu nennen seien hier beispielsweise, Festiger, Schaumfestiger, Haarsprays, Haarwachse, Haargele und andere mehr.

Beim Verbraucher sehr beliebt sind Schaumfestiger, die in der Regel auf das nasse Haar aufgetragen werden. Anschließend wird das Haar zu einer Frisur geformt (gestylt), meist mithilfe eines Föns. Die Schaumfestiger zeichnen sich dadurch aus, dass sie Polymere enthalten, die eine Fixierung der Frisur bewirken und so zum Frisurerhalt geeignet sind. Zumeist werden derartige Produkte nach jeder Haarwäsche angewandt.

Im Stand der Technik gibt es bereits Offenbarungen zu Festigerzubereitungen, die als Schaum vorliegen.

Das Dokument WO 2010/000607 A2 offenbart Zubereitungen für das Haar, die das Haar nicht-permanent formen und als Schaum vorliegen können. Diese Zubereitungen enthalten Celluloseverbindungen und ein filmbildendes und/oder festigendes Polymer.

Im Dokument WO 2006/050788 A1 werden Haarpflegezubereitung beschrieben, die schäumend sind. Diese Zubereitungen enthalten wenigstens ein Polymer, das u.a. Haar-festigend sein kann, ein kationisches Polymer, bestimmte nichtionische und zwitterionische Tenside.

Im Dokument EP 1340485 A2 werden Aerosol-Schaumzubereitungen beschrieben, die ein oberflächenaktives Tensid, ein kationisches Polymer vom Typ Vinylpyrrolidone und eine Cellulose-haltige Komponente enthalten.

Das Dokument EP 3326695 A1 offenbart einen Haarschaumfestiger mit pflegender Wirkung, der festigende Polymere, die Vinylpyrrolidone-Monomere enthalten und Cellulosemischether umfasst.

Wie aus dieser kurzen Übersicht zu erkennen ist, enthalten Haarschaum-Festiger ein oder mehrere Film-bildende/festigende Polymere, vielfach Verbindungen, die in der Regel synthetischer Natur sind. Es handelt sich beispielsweise um PVP, VP/VA-Copolymere, Polyquaternium-68, Polyquaternium-4 (quaternäres Ammoniumsalz von Hydroxyethylcellulose quaterniert mit Diallyl dimethyl ammonium Chloride) und Acrylates/Hydroxyester Acrylates Copolymere. Darüber hinaus werden in Styling-Schaumzubereitungen des Standes der Technik noch eine Vielzahl weiterer synthetischer Polymere eingesetzt.

Synthetische Polymere sind, wie der Name aussagt, synthetische Verbindungen, für die es in der Regel keine natürlichen Abbaumechanismen gibt. Die Folge ist, dass derartige Verbindungen nicht gut, sehr schlecht oder fast gar nicht biologisch abbaubar sind.

Wünschenswert ist jedoch der Einsatz von Polymeren in Styling-Schaumzubereitungen und allgemein in kosmetischen Zubereitungen, die eine gute biologische Abbaubarkeit aufweisen. Dies ist für Polymere, die natürlichen Ursprungs sind in der Regel gegeben oder deutlich besser gegeben.

Der Begriff "natürlich" im Zusammenhang mit natürlichen Inhaltsstoffen für Kosmetika ist in der ISO 16128 festgelegt. Gemäß dieser Richtlinie kann man natürliche, kosmetische Inhaltsstoffe zusammenfassend als Stoffe beschreiben, die von Pflanzen, Tieren, Mineralien oder Mikroorganismen erhältlich sind, eingeschlossen sind auch solche Stoffe, die von den genannten Quellen durch physikalische Prozesse, Fermentationsprozesse (nur solche Fermentationsprozesse, die auch in der Natur ablaufen und die zu Produkten führen, die auch in der Natur entstehen) und andere Herstellungsmethoden ohne beabsichtigte chemische Modifikation erhalten werden. Die Mikroorganismen dürfen nur solche sein, die nicht gentechnisch modifiziert wurden.

Der Begriff der biologischen Abbaubarkeit beschreibt den Prozess des Abbaus von organischen Verbindungen durch Lebewesen, insbesondere Saprobionten. Im Idealfall entstehen anorganische Stoffe, wie beispielsweise CO₂, O₂ und Ammoniak; Verbindungen, die von Pflanzen und Mikroorganismen wieder für den Aufbau von organischen Verbindungen genutzt werden.

Organische, chemische Verbindungen, die leicht biologisch abbaubar sind, sind nach OECD 301, für kosmetische Zusammensetzungen meist nach OECD 301 B klassifiziert. Diese Substanzen oder Zusammensetzungen können rasch und vollständig abgebaut werden.

Organische, chemische Verbindungen, die nach OECD 302 klassifiziert sind, sind zwar eingeschränkt, jedoch grundsätzlich biologisch abbaubar.

Die Aufgabe der vorliegenden Erfindung war es, einen Haarschaumfestiger zur Verfügung zu stellen, der Polymere enthält, die natürlichen Ursprungs sind. Die Menge an synthetischen Polymeren, die einen Beitrag zu den festigenden Eigenschaften des Haarschaumfestigers leisten, sollte verringert werden oder es sollte komplett auf derartige Polymere verzichtet werden. Die erfindungsgemäßen Haarschaumfestiger sollten jedoch in den Anwendungseigenschaften, Festigung und Frisurerhalt, mit herkömmlichen Haarschaumfestigern vergleichbar sein.

Gelöst wurden die vorstehenden Aufgaben durch ein Haarschaumfestiger, der wenigstens ein natürliches, wasserlösliches, lineares Polymer mit festigender/Film-bildender Wirkung, insbesondere Pullulan, enthält.

Im StdT sind bereits Dokumente veröffentlicht, die die Verwendung von Pullulan, offenbaren.

Das Dokument FR 3064474 B1 beschreibt die Verwendung einer Kombination von Pullulan oder Derivaten des Pullulans zusammen mit Alginsäure und/oder Hyaluronsäure zum Schutz oder zur Reparatur von Hautanhangsgebilden.

Das Dokument US 2020/0000701 A1 beschreibt Haar-Stylingzusammensetzungen, die Pullulan und wenigstens eine Cellulose-Ether-Verbindung enthalten.

Die Aufgabe der vorliegenden Erfindung war es also, einen Haarschaumfestiger zur Verfügung zu stellen, der wenigstens ein Polymer enthält, das natürlichen Ursprungs ist. Die Menge an synthetischen Polymeren, die einen Beitrag zu den festigenden/Film-bildenden Eigenschaften des Haarschaumfestigers leisten, sollte verringert werden oder es sollte komplett auf derartige Polymere verzichtet werden. Die erfindungsgemäßen Haarschaumfestiger sollten jedoch in den Anwendungseigenschaften, Festigung und Frisurerhalt, mit herkömmlichen Haarschaumfestigern vergleichbar sein.

Gelöst werden die vorstehenden Aufgaben durch ein Haarschaumfestiger enthaltend
- Pullulan in einer Menge von 0,1 bis 10,0 Gew.-%,
- wenigstens eine weitere Film-bildende Verbindung, ausgewählt aus modifizierten Stärkeverbindungen,
- optional wenigstens noch eine weitere Film- bildende Verbindung, ausgewählt aus kationisch modifizierten Guar-Verbindungen,
- optional wenigstens ein nichtionisches Tensid, insbesondere wenigstens ein Alkylpolyglucosid,
- optional wenigstens einen Cellulosemischether und
- optional PEG-40 hydrogenated castor oil.

Ebenso Gegenstand der Erfindung sind Haarschaumfestigerprodukte bestehend aus einer Aerosolpackungseinheit und einer Gesamtzusammensetzung, wobei die Gesamtzusammensetzung ein Haarschaumfestiger und ein Treibmittel ist.

Die Begriffe Haarschaumfestiger, Stylingschaum, Styling-Mousse und Schaumfestiger werden im Zusammenhang mit dieser Anmeldung synonym verwendet.

Der erfindungsgemäße Haarschaumfestiger ist eine kosmetische Zusammensetzung und bevorzugt zum Stylen des menschlichen Kopfhaares vorgesehen.

Der Wassergehalt der erfindungsgemäßen Zusammensetzungen beträgt vorteilhaft wenigstens 50 Gew.-%, bevorzugt wenigstens 55 Gew.-%, insbesondere bevorzugt 60 Gew.-%, bezogen auf das Gesamtgewicht des Haarschaumfestigers. Der Wassergehalt der erfindungsgemäßen Zusammensetzungen beträgt vorteilhaft höchstens 95 Gew.-%, bezogen auf das Gesamtgewicht des Haarschaumfestigers.

Das natürliche, wasserlösliche, lineare Polymer mit festigender/Film-bildender Wirkung ist Pullulan. Pullulan ist ein α-1,6 verknüpftes Polysaccharid aus Maltotriose-Einheiten, das aus dem Pilz *Aureobasidium pullulans* gewonnen werden kann. Die Glucose-Einheiten der Maltotriose sind α-1,4-glycosidisch miteinander verknüpft. Pullulan ist gut in Wasser löslich.

Pullulan kann beispielsweise unter dem Handelsnamen Pullulan von der Firma Hayashibara Co., LTD. bezogen werden.

In dem erfindungsgemäßen Haarschaumfestiger ist Pullulan mit einem Gehalt von 0,2 bis 8,0 Gew.-%, bevorzugt von 0,5 bis 5,0 Gew.-% enthalten, bezogen auf das Gesamtgewicht des Haarschaumfestigers.

Der erfindungsgemäße Haarschaumfestiger enthält wenigstens eine weitere Film-bildende Verbindung. Diese Verbindung wird aus gewählt aus Polymeren natürlichen Ursprungs, insbesondere aus der Gruppe der Stärkeverbindungen, die modifiziert sind. Die Stärkeverbindungen können aus verschiedenen Quellen stammen, beispielsweise Reisstärke, Maisstärke oder Kartoffelstärke. Die Gruppe der modifizierten Stärkeverbindungen hat auch verdickende Eigenschaften. Im Zusammenhang mit der vorliegenden Erfindung sind jedoch die Film-bildenden Eigenschaften relevant und tragen zur Erfindung bei. Die erfindungsgemäßen Stärkeverbindungen können nichtionische Biopolymere sein oder einen ionischen Charakter haben. Stärke ist ein Speicherkohlenhydrat der Pflanzen, das aus zwei Molekültypen besteht, Amylose und Amylopektin. Kennzeichnend für Amylose sind lineare helikale Ketten aus α-1,4-glykosidisch verknüpften Glukosemolekülen, während Amylopektin eine verzweigte Struktur aufweist. Diese Verzweigung wird durch zusätzliche α-1,6 glykosidisch verknüpfte Glukosemoleküle erreicht.

Erfindungsgemäß bevorzugt ist der Einsatz wenigstens einer modifizierten Maisstärkeverbindung. Die Begriffe Maisstärkeverbindung und Maisstärke werden im Zusammenhang mit dieser Erfindung synonym verwendet.

Modifizierte Maisstärke kann beispielsweise als Hydroxypropylstärke vorliegen, die INCI-Bezeichnung lautet Corn Starch Modified. Eine derartige Verbindung ist beispielsweise bei der Firma Nouryon unter der Handelsbezeichnung Amaze erhältlich. Weiterhin kann die Stärke auch als hydrolysierte Stärke vorliegen, dabei wird die Stärke meist durch eine Säurebehandlung, aber auch enzymatisch, zu Zwischenprodukten abgebaut. Derartige Produkte haben die INCI-Bezeichnung Hydrolyzed Corn Starch (erhältlich beispielsweise als MaizeCare Style Polymer bei der Firma Dow) oder Maltodextrin (erhältlich beispielsweise als Agenanova 30.326 von der Firma Agrana). Darüber hinaus kann die modifizierte Maisstärke auch als Distärkephosphat vorliegen, erhältlich beispielsweise als Agenajel 20.306 von der Firma Agrana. Diese Verbindung hat die INCI-Bezeichnung Distarch Phosphate. Eine Kombination aus hydroxypropylierter Stärke mit phosphatierter Stärke hat die INCI-Bezeichnung Hydroxyproyl Starch Phosphate und ist beispielsweise unter Bezeichnung Agenajel 20.383 Bei der Firma Agrana erhältlich.

Modifizierte Kartoffelstärke kann beispielsweise als hydroxypropylierte Stärke vorliegen; diese Verbindung hat die INCI-Bezeichnung Hydroxypropyl Starch und ist beispielsweise erhältlich bei der Firma Agrana unter der Handelsbezeichnung Amitrolit 8850.

Modifizierte Reisstärke kann beispielsweise als Distärkephosphat vorliegen; diese verbindung hat die INCI-Bezeichnung Distarch Phosphate und ist beispielsweise mit der Handelsbezeichnung Rice PO4 Natural bei der Firma Agrana erhältlich.

Bevorzugt ist der Einsatz einer hydroxypropylierten Maisstärke mit der INCI-Bezeichnung Corn Starch Modified.

Die wenigstens eine weitere Film-bildende Verbindung ausgewählt aus der Gruppe der modifizierten Stärkeverbindungen liegt mit einem Gesamtgehalt von 0,1 bis 8,0 Gew. %, bevorzugt von 0,5 bis 5,0 Gew. % vor, bezogen auf das Gesamtgewicht des Haarschaumfestigers.

Der erfindungsgemäße Haarschaumfestiger kann optional wenigstens noch eine weitere Film- bildende Verbindung ausgewählt aus kationisch modifizierten Guar-Verbindungen enthalten. Dazu wird beispielsweise Guarkernmehl zunächst mit Propylenoxid verethert und anschließend weiter modifiziert zu Guar Hydroxypropyltrimonium Chlorid. Guar Hydroxypropy-Itrimonium Chlorid hat neben seiner Film-bildenden Wirkung auch einen antistatischen, Viskositäts-regulierenden sowie Haut- und Haar-pflegenden Effekt.

Guar Hydroxypropyltrimonium Chlorid ist mit verschiedenen Molekulargewichten und mit verschiedenen Ladungsdichten erhältlich. Als vorteilhaft haben sich Guar Hydroxypropyltrimonium Chlorid-Verbindungen mit einem Molekulargewicht von etwa 1,0 bis 3,0 Million Dalton erwiesen. Ebenso haben sich Guar Hydroxypropyltrimonium Chlorid-Verbindungen mit einer Ladungsdichte von 0,5 bis 1,0 mmol/g als vorteilhaft erwiesen.

Die Bestimmung des Molekulargewichts kann vorteilhaft mit der Methode der Gel-Permeations-Chromatographie bestimmt werden.

Die Ladungsdichte eines Polymers und in Bezug auf Guar Hydroxypropyltrimonium Chlorid-Verbindungen die kationische Ladungsdichte, bezieht sich auf das Verhältnis der Anzahl positiver Ladungen auf einem Polymer zum Molekulargewicht des Polymers. Die kationische Ladungsdichte multipliziert mit dem Molekulargewicht des Polymers bestimmt die Anzahl der positiv geladenen Stellen auf einer gegebenen Polymerkette. Die Ladungsdichte kann auch definiert werden als die Anzahl der Milliäquivalente der Ladung (quaternärer Stickstoff) pro Gramm (meq/g) des Polymers.

Bei Guar Hydroxypropyltrimonium Chlorid-Verbindungen, die als kationische Guare bezeichnet werden können, kann der Substitutionsgrad, d.h. die Anzahl der durch kationische Gruppen substituierten Hydroxylgruppen an einer bestimmten Zuckereinheit (maximal drei pro Zucker) bestimmt werden. Der Substitutionsgrad wird verwendet, um die N-Äquivalente pro Zuckerteil zu berechnen, was dann zu den Milliäquivalenten der Ladung pro Gramm Polymer führt.

Guar Hydroxypropyltrimonium Chlorid kann beispielsweise von der Firma BASF unter der Handelsbezeichnung Dehyquart Guar N oder unter der Handelsbezeichnung Dehyquart Guar TC bezogen werden.

Wenn in dem erfindungsgemäßen Haarschaumfestiger wenigstens eine kationisch modifizierte Guar-Verbindung enthalten ist, so liegt diese Verbindung vorteilhaft mit einem Gesamtgehalt von 0,01 Gew.-% bis 2,0 Gew.-%, bevorzugt von 0,05 Gew.-% bis 1,5 Gew.-%, insbesondere bevorzugt von 0,1 Gew.-% bis 1,0 Gew.-% vor, bezogen auf das Gesamtgewicht des Haarschaumfestigers und den Aktivgehalt.

In der Haarschaumfestiger-Zubereitung kann optional wenigstens eine Cellulosemischether-Verbindung enthalten sein. Cellulosemischether können unter den Oberbegriff Celluloseverbindungen eingeordnet werden. Unter Celluloseverbindungen werden alle Polysaccharidverbindungen verstanden, die in ihrer Struktur Verknüpfungen von Glucoseresten enthalten, die über β-1,4-Bindungen verknüpft sind. Neben den unsubstituierten Cellulosen können die Cellulosederivate anionisch, kationisch, amphoter oder nichtionisch sein. Von diesen Derivaten sind die Celluloseether, Celluloseester und Celluloseetherester zu unterscheiden. Unter den Celluloseestern gibt es anorganische Celluloseester (Nitrate, Sulfate oder Phosphate von Cellulose ...), organische Celluloseester (Monoacetate, Triacetate, Amidopropionate, Acetatbutyrate, Acetatpropionate oder Acetattrimellitate von Cellulose ...) und gemischte organische/anorganische Ester von Cellulose, wie Acetatbutyratsulfate und Acetatpropionatsulfate von Cellulose. Von den Celluloseetherestern können die Phthalate von Hydroxypropylmethylcellulose und die Sulfate von Ethylcellulose angegeben werden.

Die erfindungsgemäßen Celluloseverbindungen sind unter den nichtionischen Celluloseethern ausgewählt. Die nichtionischen Celluloseether umfassen Alkylcellulosen, wie beispielsweise Methylcellulosen und Ethylcellulosen; Hydroxyalkylcellulosen, wie beispielsweise Hydroxymethylcellulosen, Hydroxyethylcellulosen und Hydroxypropylcellulosen; gemischte Hydroxyalkyl-alkylcellulosen, die auch als Cellulosemischether bezeichnet werden. *Cellulosemischether* sind Verbindungen wie beispielsweise Hydroxypropylmethylcellulosen, Hydroxyethylmethylcellulosen, Hydroxyethylethylcellulosen und Hydroxybutylmethylcellulosen.

Bevorzugt sind Hydroxypropylmethylcellulosen, geeignete Hydroxypropylmethylcellulosen können beispielsweise unter der Handelsbezeichnung Methocel 40-202 PCG bei der Dow Chemical Comp. bezogen werden.

Hydroxypropylmethylcellulosen können durch die folgende Strukturformel beschrieben werden:

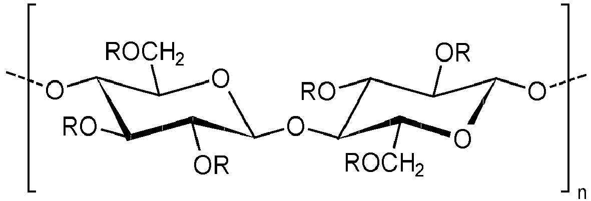

R steht für-H, -CH₃, -CH₂-CHOH-CH₃.

Wenn in dem erfindungsgemäßen Haarschaumfestiger wenigstens ein Cellulosemischether enthalten ist, so liegt der wenigstens eine Cellulosemischether vorteilhaft mit einem Gesamtgehalt von 0,02 Gew.-% bis 2,0. Gew.-%, bevorzugt von 0,05 Gew.-% bis 1,0 Gew.-%, insbesondere bevorzugt von 0,1 Gew.-% bis 0,5 Gew.-% vor, bezogen auf das Gesamtgewicht des Haarschaumfestigers und den Aktivgehalt.

Unter den geeigneten Hydroxypropylmethylcellulosen sind bevorzugt Verbindungen auszuwählen, die eine kalte Verarbeitung, d.h. eine Verarbeitung bei 15 bis 25 °C erlauben.

Wenn nicht vollständig auf synthetische Polymere, insbesondere auf festigende, synthetische Polymere verzichtet werden soll, so können vorteilhaft Polymere ausgewählt werden, die Vinylpyrrolidone Monomere enthalten.

Dies können zum einen Mischpolymerisate aus Vinylpyrrolidone und Vinylacetat, beispielsweise VP/VA Copolymere. Derartige VP/VA Copolymere sind beispielsweise erhältlich unter der Handelsbezeichnung Luviskol VA 64 W von der Firma BASF.

Ebenso vorteilhaft sind auch Homopolymere aus Vinylpyrrolidone (PVP) sein, beispielsweise erhältlich als Luviskol K 30 Super P (100%) von der Firma BASF.

Festigende Polymere bleiben nach dem Verdunsten des Lösungsmittels (oft Wasser und/oder Ethanol) als Film auf dem Haar haften und fixieren dessen Form.

Wenn in dem erfindungsgemäßen Haarschaumfestiger wenigstens ein Polymer enthaltend Vinyl-Pyrrolidon Monomere enthalten ist, so liegt dieses wenigstens eine Polymer vorteilhaft mit einem Gesamtgewicht von 0,5 Gew.-% bis 20,0 Gew.-%, bevorzugt von 1,0 Gew.-% bis 10,0 Gew.-%, insbesondere bevorzugt von 1,5 Gew.-% bis 8,0 Gew.-%, bezogen auf das Gesamtgewicht des Haarschaumfestigers und den Aktivgehalt, vor.

Der erfindungsgemäße Haarschaumfestiger kann optional wenigstens ein nichtionisches Tensid enthalten. Nicht-ionische Tenside bilden in wässrigem Medium keine Ionen.

Es ist bevorzugt, wenn das wenigstens eine nichtionische Tensid ausgewählt wird aus Alkylpolyglucosiden, insbesondere aus der Gruppe Laurylglucosid, Decylglucosid, Cocoglucosid und/oder Caprylyl/Capryl Glucoside.

Wenn in dem erfindungsgemäßen Haarschaumfestiger wenigstens ein nichtionisches Tensid enthalten ist, so liegt dieses wenigstens eine nichtionische Tensid vorteilhaft mit einem Gesamtwicht von 0,1 bis 2,0 Gew.-%, bevorzugt 0,2 bis 1,0 Gew.-% vor, bezogen auf das Gesamtgewicht des Haarschaumfestigers und den Aktivgehalt.

In dem erfindungsgemäßen Haarschaumfestiger kann optional PEG-40 gehärtetes Rizinusöl (PEG 40 hydrogenated castor oil) enthalten sein. PEG-40 hydrogented castor oil hat bedingt durch seinen amphiphilen Aufbau mehrere Funktionen, zu einen kann es als Emulgator und Lösungsvermittler (Lösungsmittel) wirken und zum anderem auch als Emolliens.

Wenn in der erfindungsgemäßen Zubereitung PEG-40 hydrogenated castor oil enthalten ist, so liegt es mit einem Gesamtgewicht von 0,1 bis 2,0 Gew. %, bevorzugt 0,2 bis 1,0 Gew.-% vor, bezogen auf das Gesamtgewicht der Zubereitung.

In einer möglichen Ausführungsform, wenn nicht komplett auf den Einsatz von synthetischen Polymeren verzichtet werden soll, wird ein Haarschaumfestiger bereitgestellt, der
- Pullulan in einer Menge von 0,1 bis 10,0 Gew.-%,
- wenigstens eine weitere Film-bildende Verbindung, ausgewählt aus modifizierten Stärkeverbindungen,
- wenigstens ein festigendes Polymer enthaltend Vinylpyrrolidone-Monomere,
- optional wenigstens noch eine weitere Film- bildende Verbindung, ausgewählt aus kationisch modifizierten Guar-Verbindungen,
- optional wenigstens ein nichtionisches Tensid, insbesondere wenigstens ein Alkylpolyglucosid,
- optional wenigstens einen Cellulosemischether und
- optional PEG-40 hydrogenated castor oil
umfasst.

In einer weiteren Ausführungsform wird ein Haarschaumfestiger bereitgestellt, der
- Pullulan in einer Menge von 0,1 bis 10,0 Gew.-%,
- wenigstens eine weitere Film-bildende Verbindung, ausgewählt aus modifizierten Stärkeverbindungen,
- wenigstens einen Cellulosemischether,
- wenigstens ein nichtionisches Tensid, wobei das wenigstens eine nichtionische Tensid ein Alkylpolyglucosid ist und
- PEG-40 hydrogenated castor oil
umfasst.

In einer vorteilhaften Ausführungsform wird ein Haarschaumfestiger bereitgestellt, der
- Pullulan in einer Menge von 0,1 bis 10,0 Gew.-%,
- wenigstens eine weitere Film-bildende Verbindung, ausgewählt aus modifizierten Stärkeverbindungen,
- wenigstens eine weitere Film- bildende und/oder festigende Verbindung, wobei die wenigstens eine weitere Film- bildende und/oder festigende Verbindung eine kationisch modifizierte Guar-Verbindung ist,
- wenigstens ein nichtionisches Tensid, wobei das wenigstens eine nichtionische Tensid ein Alkylpolyglucosid ist,
umfasst.

Verbindungen, mit denen der pH-Wert auf einen gewünschten Wert eingestellt werden kann, können vorteilhaft verwendet werden. Geeignet sind alle Verbindungen, die in der kosmetischen Industrie üblich sind, bevorzugt werden jedoch die Verbindungen Citronensäure, Milchsäure und/oder Natronlauge eingesetzt. Der pH-Wert des erfindungsgemäßen Haarschaumfestigers liegt vorteilhaft im Bereich von 4,2 bis 5,4.

Erfindungsgemäß vorteilhaft ist ebenfalls die Verwendung einer oder mehrerer Substanzen der Vitamine der B Gruppe und/oder des Vitamin B-Komplexes, die den erfindungsgemäßen Zusammensetzungen zusätzlich zugesetzt werden können. Substanzen der Vitamin B Gruppe und/oder des Vitamin B-Komplexes sind üblicherweise wasserlöslich und spielen insbesondere für den Zellmetabolismus bei Pflanzen und Tieren eine besondere Rolle.

Beispiele erfindungsgemäßer Substanzen der Vitamine der B Gruppe und/oder des Vitamin B-Komplex sind unter anderem Thiamin (Vitamin B1), Riboflavin (Vitamin B2), Nicotinsäure (Vitamin B3), Nicotinsäureamid (Niacinamid), Pantothensäure (Vitamin B5), Panthenol (Provitamin B5), Panthenoltriacetat, Panthenolmonoethylether, Pantolacton, Pyridoxin und Pyridoxal.

Bevorzugt ist die Verwendung von Panthenol als Substanz der Vitamine der B Gruppe und/oder des Vitamin B-Komplexes in den erfindungsgemäßen Zusammensetzungen.

Panthenol kann beispielsweise unter dem Handelsnamen D-Panthenol 75 W als 77% Lösung in Wasser von der BASF bezogen werden.

Eine oder mehrere Substanzen der Vitamine der B Gruppe und/oder des Vitamin B-Komplex sind erfindungsgemäß in einem Anteil von 0,005 Gew.-% bis 5 Gew.-%, bevorzugt 0,05 Gew.-% bis 2 Gew.-%, insbesondere bevorzugt 0,01 Gew.-% bis 1,0 Gew.-%, bezogen auf das Gesamtgewicht des Haarschaumfestigers und den Aktivgehalt, in den erfindungsgemäßen Zusammensetzungen enthalten.

Die erfindungsgemäßen Zusammensetzungen können konserviert werden. Dazu können alle Konservierungsstoffe verwendet werden, die gemäß Kosmetikverordnung verwendet werden dürfen. Bevorzugt ist jedoch eine Konservierung mit Benzoesäure und/oder ihren Salzen.

Die jeweiligen Konservierungsstoffe können einzeln oder in Kombination eingesetzt werden. Die Gesamtmenge an einem Konservierungsstoff oder mehreren Konservierungsstoffen wird vorteilhaft gewählt aus dem Bereich von 0,01 bis 1,0 Gew.-%, bevorzugt 0,1 bis 0,8 Gew.-%, bezogen auf das Gesamtgewicht des Haarschaumfestigers und den Aktivgehalt.

Das Schaumfestigerprodukt umfasst eine Verpackungseinheit und eine Gesamtzusammensetzung, die vortei
Ihaft wiederum aus dem Haarschaumfestiger und einem Treibmittel besteht.

Die Verpackungseinheit ist erfindungsgemäß vorteilhaft ein Aerosolbehälter mit Sprühvorrichtungen. Die Aerosolbehälter enthalten die Schaumfestigerzusammensetzung und das Treibmittel. Aerosolbehälter stehen unter dem Druck des Treibmittels. Derartige Behälter können mit Ventilen sehr unterschiedlicher Bauart ausgestattet sein, die ein Aufschäumen bei der Entnahme des Inhalts ermöglichen.

Als Druckgasbehälter kommen im Sinne der vorliegenden Erfindung vor allem zylindrische Gefäße aus Metall (Aluminium, Weißblech, Inhalt <1000 ml) in Frage, bei deren Auswahl Druck- und Bruchfestigkeit, Korrosionsbeständigkeit, leichte Füllbarkeit, usw., aber auch ästhetische Gesichtspunkte, Handlichkeit, Bedruckbarkeit etc. eine Rolle spielen. Der maximale zulässige Betriebsdruck von Sprüh-Dosen aus Metall bei 50°C ist 15 bar 18 bar für Haarsprays; das maximale Füllvolumen bei dieser Temperatur liegt bei ca. 90 % des Gesamtvolumens.

Besonders vorteilhaft im Sinne der vorliegenden Erfindung sind Dosen aus Weißblech oder Aluminium. Aus Korrosionsschutzgründen können Metalldosen innen lackiert sein, wozu alle handelsüblichen Innenschutzlacke geeignet sind. Bevorzugt im Sinne der vorliegenden Erfindung sind Polyester-, Epoxyphenol- sowie Polyamidimidlacke. Auch Folienkaschierungen aus Polyethylen (PE), Polypropylen (PP) und/oder Polyethylenterephthalat (PET) im Innern der Dosen sind vorteilhaft, insbesondere für Dosen aus Weißblech.

Die Druckgasbehälter sind üblicherweise ein- oder zwei-, meist aber dreiteilig zylindrisch, konisch oder anders geformt.

Der innere Aufbau der Sprüh-Dosen sowie die Ventilkonstruktion sind je nach VerwendungsZweck und der physikalischen Beschaffenheit des Inhalts - z. B. ob als Zwei- oder als Dreiphasensystem - sehr variantenreich und können vom Fachmann durch einfaches Ausprobieren ohne erfinderisches Zutun ermittelt werden. Für geeignete Ausführungsformen sei auf das "Aerosol Technologie Handbuch der Aersosol-Verpackung" hingewiesen (*Wolfgang Tauscher, Melcher Verlag GmbH Heidelberg*/*München, 1996).*

Erfindungsgemäß vorteilhafte Ventile können mit oder ohne Steigrohr ausgebildet sein. Die Einzelteile, aus welchen erfindungsgemäße Ventile üblicherweise aufgebaut sind, bestehen vorzugsweise aus den folgenden Materialien:
- Teller:: Weißblech: blank, ggf. gold- bzw. klarlackiert (PE, PP oder PET) Aluminium: blank, silber- oder goldlackiert, verschiedene Lackvarianten, Stoner-Mudge-Ausführung.
- Dichtung:: natürliche bzw. synthetische Elastomere bzw. thermoplastische (Sleeve-Gaskets, folienkaschiert aus PE oder PP) Innen- und Außendichtungen, z. B. aus Perbunan, Buna, Neopren, Butyl, CLB, LDPE, Viton, EPDM, Chlorbutyl, Brombutyl und/oder diversen Compounds.
- Kegel:: Polyamid (PA), Polyoxymethylen (POM), Messing sowie diversen Sonder-materialen, Standardbohrungen (z. B.: 0,25 bis 0,70 mm oder 2 x 0,45 bis 2 x 1,00 mm), verschiedene Schaftdurchmesser
- Feder:: Metall, besonders bevorzugt V2A, rostfreier Stahl; Kunststoff und auch Elastomer.
- Gehäuse:: Standard und Impact VPH-Bohrungen, RPT-Bohrungen oder geschlitzt für Überkopf-Anwendungen Materialien: z. B. Polyacetal, PA, PE, POM und dergleichen mehr
- Steigrohr:: Kunststoff (Polymer Resin), z.B. PE, PP, PA oder Polycarbonat.

Vorteilhafte Sprühköpfe im Sinne der vorliegenden Erfindung sind beispielsweise Schaumköpfe für die aufrechte Anwendung (Dose senkrecht halten) oder Schaumköpfe für die Überkopf-Anwendung mit einem oder mehreren Kanälen.

Es ist erfindungsgemäß bevorzugt, wenn der Aerosolbehälter eine Aluminiumdose ist, die auf der Innenseite mit einem Schutzlack, insbesondere mit Polyurethan oder einem Polyesterlack, beschichtet ist. Die genannten Beschichtungen sind nicht Epoxidharz-basiert und werden somit als BPA-ni klassifiziert. BPA-ni bedeutet, dass die Ausgangssubstanz nicht Bisphenol A ist, eine Substanz, die wegen einer möglichen östrogenen Wirkung in der öffentlichen Diskussion steht.

Gleichwohl sind aber auch Epoxyphenollacke als Innenschutzlacke möglich.

Die Treibgase des Treibmittels können aus Kohlenwasserstoffgasen, fluorierten Gasen, Fluorkohlenwasserstoffgase, Dimethylether, Stickstoff, Luft oder Kohlendioxid oder Mischungen davon ausgewählt werden. Bevorzugt sind Treibmittel, die eine Mischung aus den Treibgasen Isobutan, Propan und n-Butan sind.

Die bevorzugte Druckgasstufe wird vorteilhaft aus dem Bereich von 2,4 bis 3,5 bar ausgewählt.

Vorteilhaft im Sinne der vorliegenden Erfindung wird der Gewichtsanteil an Treibmittel aus dem Bereich von 15,0 bis 1,0 Gew.-%, insbesondere 12,0 bis 2,0 Gew.-%, bezogen auf das Gesamtgewicht der Gesamtzusammensetzung, die aus Haarschaumfestiger und Treibmittel besteht, gewählt. Der Haarschaumfestiger liegt demnach mit einem Gewichtsanteil von 99 bis 85 Gew.-%, insbesondere 98 bis 88 Gew.-% in der Gesamtzusammensetzung vor.

Die erfindungsgemäßen Haarschaumfestiger liegen vorteilhaft in Form von verschäumbaren oder nachschäumbaren Zusammensetzungen vor, welche aus Aerosolbehältern entnommen werden und beim Austritt aus der Düse aufschäumen. Erfindungsgemäß vorteilhafte Aerosolbehälter sind Sprühvorrichtungen mit einer Füllung aus einem weitgehend flüssigen Haarschaumfestiger, der unter dem Druck eines Treibmittels steht. Derartige Behälter können mit Ventilen sehr unterschiedlicher Bauart ausgestattet sein, die ein Aufschäumen bei der Entnahme des Inhalts ermöglichen.

Um zu zeigen, dass die Merkmale des Gegenstandes der Erfindung, nämlich ein Haarschaumfestiger enthaltend Pullulan in einer Menge von 0,1 bis 10,0 Gew.-% und wenigstens eine weitere Film-bildende Verbindung, ausgewählt aus modifizierten Stärkeverbindungen, einen Beitrag zur Lösung der Aufgaben leisten, wurden verschiedene Haarschaumfestiger (Tabelle 1, Beispiele 1 bis 5) hergestellt. Ein Haarschaumfestiger gemäß Beispiel 1 enthielt Polyquaternium-68, VP/VA-Polymer und Hydroxypropyl Methylcellulose, eine Kombination, die die Film-Bildung auf dem Haar, die Festigung des Haares und Verdickung der Zubereitung bewirkt. Die beiden erst genannten Polymere sind synthetischer Natur. Hydroxypropyl Methylcellulose ist eine modifizierte Cellulosverbindung.; Eine Zubereitung gemäß Beispiel 1 dient als Vergleichszubereitung, die den StdT widerspiegelt. Ein Haarschaumfestiger gemäß Beispiel 5 enthält neben Pullulan ein synthetisches festigendes Polymer, nämlich VP/VA-Copolymer. Dieses Ausführungsbeispiel beschreibt eine Ausführungsform, bei der nicht vollständig auf synthetische Polymere verzichtet wurde. Die Beispiele 2 bis 4 illustrieren Ausführungsformen, die natürliche und oder natur-basierte Komponenten enthalten.

Jeweils 0,3 g des Haarschaumfestigers gemäß den genannten Beispielen wurden auf 3 g schwere, handtuchtrockene, gekämmte Haartressen homogen aufgetragen. Jeweils der erfahrene Labormitarbeiter haben die Bewertung durchgeführt. Der Haarschaumfestiger wurde in Bezug auf das Schaumverhalten und das Verhalten beim Auftragen auf das Haar beurteilt.

Der Zustand des getrockneten Haares wurde beurteilt, und zwar in Bezug auf die Halteeigenschaften (wann bricht der Film auf den Haaren zwischen den Fingern), Halt, Festigung und Klebrigkeit.

Es wurde deutlich, dass mit dem Einsatz von natürlichen Komponenten in Haarschaumfestigern die Halteeigenschaften gegenüber der Vergleichszubereitung (Beispiel 1) verbessert werden können. Auch die übrigen o.g. Eigenschaften und die Sensorik bei der Applikation wurden für alle Zubereitungen als gut bewertet.

Somit konnte gezeigt werden, dass mit der Einarbeitung von natürlichen festigenden und/oder Film-bildenden Polymeren gleiche Anwendungseigenschaften erzielt werden, wie sie mit synthetischen Polymeren erreicht werden. In Bezug auf die Halteeigenschaften konnte sogar eine Verbesserung erzielt werden.

### Beispiele:

Nachfolgende Beispiele sollen die Erfindung verdeutlichen, ohne sie einzuschränken. Dabei geben die angeführten Mengen die Konzentrationen der Rohstoffe an, nicht den Aktivgehalt, jeweils in Gewichtsprozent, sofern keine anderen Angaben gemacht wurden.

| **INCI (Aktivgehalt)** | 1 | 2 | 3 | 4 | 5 | 6 | 7 |
|---|---|---|---|---|---|---|---|
| Aqua | 94,69 | 94,69 | 95,49 | 94,69 | 93,49 | 94,39 | 94,99 |
| VP/VA Copolymer | 3,5 | | | | 3,5 | | |
| Polyquaternium-68 | 0,5 | | | | | | |
| Hydroxypropyl Methylcellulose | 0,3 | 0,3 | | 0,3 | | | |
| Pullulan | | 4 | 2,5 | 2 | 1 | 2 | 1,5 |
| Corn Starch Modified | | | | 2 | 1 | 2 | 2 |
| Guar Hydroxypropyltrimonium Chloride | | | 1 | | | | 0,5 |
| Citric Acid | 0,1 | 0,1 | 0,1 | 0,1 | 0,1 | 0,1 | 0,1 |
| Sodium Hydroxide | 0,01 | 0,01 | 0,01 | 0,01 | 0,01 | 0,01 | 0,01 |
| Sodium Benzoate | 0,25 | 0,25 | 0,25 | 0,25 | 0,25 | 0,25 | 0,25 |
| PEG-40 Hydrogenated Castor Oil | 0,4 | 0,4 | | 0,4 | | | |
| Decyl Glucoside | | | 0,4 | | | | |
| Caprylyl/Capryl Glucoside | | | | | 0,4 | 0,4 | 0,4 |
| Parfum | 0,25 | 0,25 | 0,25 | 0,25 | 0,25 | 0,25 | 0,25 |
| | | | | | | | |
| Halteeigenschaften | o | o | + | + bis ++ | ++ bis +++ | + bis ++ | + |

## Patentansprüche

1. Haarschaumfestiger enthaltend
• Pullulan in einer Menge von 0,1 bis 10,0 Gew.-%,
• wenigstens eine weitere Film-bildende Verbindung ausgewählt aus modifizierten Stärkeverbindungen,
• optional wenigstens noch eine weitere Film- bildende Verbindung, ausgewählt aus kationisch modifizierten Guar-Verbindungen,
• optional wenigstens einen Cellulosemischether und
• optional wenigstens ein nichtionisches Tensid, insbesondere wenigstens ein Alkylpolyglucosid, und/oder PEG-40 gehärtetes Rizinusöl.

2. Haarschaumfestiger nach Anspruch 1 **dadurch gekennzeichnet, dass** der Wassergehalt wenigstens 50 Gew.-%, bevorzugt wenigstens 55 Gew.-%, insbesondere bevorzugt wenigstens 60 Gew.-% und höchstens 95 Gew.-%, bezogen auf das Gesamtgewicht des Haarschaumfestigers.

3. Haarschaumfestiger nach Anspruch 1 und/oder 2, **dadurch gekennzeichnet, dass** Pullulan mit einem Gehalt von 0,2 bis 8,0 Gew.-%, bevorzugt von 0,5 bis 5,0 Gew.-% enthalten, bezogen auf das Gesamtgewicht des Haarschaumfestigers.

4. Haarschaumfestiger nach wenigstens einem der vorstehenden Ansprüche, **dadurch gekennzeichnet, dass** die wenigstens eine modifizierte Stärkeverbindung ausgewählt wird aus modifizierter Maisstärke, modifizierter Reisstärke und/oder modifizierter Kartoffelstärke, insbesondere modifizierte Maisstärke.

5. Haarschaumfestiger nach wenigstens einem der vorstehenden Ansprüche, **dadurch gekennzeichnet, dass** die wenigstens eine modifizierte Stärkeverbindung mit einem Gesamtgehalt von 0,1 bis 8,0 Gew.-%, bevorzugt von 0,5 bis 5,0 Gew.-% vorliegt, bezogen auf das Gesamtgewicht des Haarschaumfestigers.

6. Haarschaumfestiger nach wenigstens einem der vorstehenden Ansprüche, **dadurch gekennzeichnet, dass**
• Pullulan in einer Menge von 0,1 bis 10,0 Gew.-%,
• wenigstens eine weitere Film-bildende Verbindung ausgewählt aus modifizierten Stärkeverbindungen,
• wenigstens ein festigendes Polymer enthaltend Vinylpyrrolidone-Monomere,
• optional wenigstens noch eine weitere Film- bildende Verbindung, ausgewählt aus kationisch modifizierten Guar-Verbindungen,
• optional wenigstens einen Cellulosemischether und
• wenigstens ein nichtionisches Tensid, insbesondere wenigstens ein Alkylpolyglucosid, und/oder PEG-40 gehärtetes Rizinusöl
enthalten ist.

7. Haarschaumfestiger nach wenigstens einem der Ansprüche, 1 bis 5, **dadurch gekennzeichnet, dass**
• Pullulan in einer Menge von 0,1 bis 10,0 Gew.-%,
• wenigstens eine weitere Film-bildende Verbindung ausgewählt aus modifizierten Stärkeverbindungen,
• wenigstens ein Cellulosemischether,
• wenigstens ein nichtionisches Tensid, insbesondere wenigstens ein Alkylpolyglucosid und/oder PEG-40 gehärtetes Rizinusöl
enthalten ist.

8. Haarschaumfestiger nach wenigstens einem der Ansprüche 1 bis 5, **dadurch gekennzeichnet, dass**
• Pullulan in einer Menge von 0,1 bis 10,0 Gew.-%,
• wenigstens eine weitere Film-bildende Verbindung ausgewählt aus modifizierten Stärkeverbindungen,
• wenigstens noch eine weitere Film- bildende Verbindung, wobei die wenigstens noch eine weitere Film- bildende Verbindung eine kationisch modifizierte Guar-Verbindung ist und
• wenigstens ein nichtionisches Tensid, insbesondere wenigstens ein Alkylpolyglucosid, und/oder PEG-40 gehärtetes Rizinusöl
enthalten ist.

9. Haarschaumfestiger nach wenigstens einem der Ansprüche 1 bis 5 und Anspruch 7, **dadurch gekennzeichnet, dass** der wenigstens eine Cellulosemischether ausgewählt wird aus Hydroxypropylmethylcellulosen, Hydroxyethyl-methylcellulosen, Hydroxyethylethylcellulosen und Hydroxybutylmethylcellulosen, insbesondere aus Hydroxypropylmethylcellulosen.

10. Haarschaumfestiger nach wenigstens einem der Ansprüche 1 bis 5 und/oder Anspruch 7 und 9, **dadurch gekennzeichnet, dass** der wenigstens ein Cellulosemischether mit einem Gesamtgehalt von 0,02 Gew.-% bis 2,0 Gew.-%, bevorzugt von 0,05 Gew.-% bis 1,0 Gew.-%, insbesondere bevorzugt von 0,1 Gew.-% bis 0,5 Gew.-% vorliegt, bezogen auf das Gesamtgewicht des Haarschaumfestigers und den Aktivgehalt.

11. Haarschaumfestiger nach wenigstens einem der Ansprüche 1 bis 5 und Anspruch 8, **dadurch gekennzeichnet, dass** die wenigstens noch eine weitere Film- bildende Verbindung, ausgewählt aus wenigstens einer kationisch modifizierten Guar-Verbindung, weiter insbesondere Guar Hydroxypropyltrimonium Chlorid ist.

12. Haarschaumfestiger nach wenigstens einem der Ansprüche 1 bis 5 und/oder Anspruch 8 und 11, **dadurch gekennzeichnet, dass** die wenigstens eine kationisch modifizierte Guar-Verbindung mit einem Gesamtgehalt von 0,01 Gew.-% bis 2,0 Gew.-%, bevorzugt von 0,05 Gew.-% bis 1,5 Gew.-%, insbesondere bevorzugt von 0,1 Gew.-% bis 1,0 Gew.-% vorliegt, bezogen auf das Gesamtgewicht des Haarschaumfestigers und den Aktivgehalt.

13. Haarschaumfestiger nach wenigstens einem der Ansprüche 1 bis 8, **dadurch gekennzeichnet, dass** das wenigstens eine nichtionische Tensid ausgewählt aus Alkylpolyglucosiden, aus der Gruppe Laurylglucosid, Decylglucosid, Cocoglucosid und/oder Caprylyl/Capryl Glucoside ausgewählt wird.

14. Haarschaumfestiger nach wenigstens einem der Ansprüche 1 bis 8 und/oder Anspruch 13, **dadurch gekennzeichnet, dass** das wenigstens eine nichtionische Tensid ausgewählt aus Alkylpolyglucosiden mit einem Gesamtgehalt von 0,1 bis 2,0 Gew.-%, bevorzugt 0,2 bis 1,0 Gew.-% vorliegt, bezogen auf das Gesamtgewicht des Haarschaumfestigers und den Aktivgehalt.

15. Haarschaumfestiger nach wenigstens einem der Ansprüche 1 bis 8, **dadurch gekennzeichnet, dass** PEG-40 gehärtetes Rizinusöl mit einem Gehalt von 0,1 bis 2,0 Gew. %, bevorzugt 0,2 bis 1,0 Gew. % vorliegt, bezogen auf das Gesamtgewicht der Zubereitung.

16. Haarschaumfestigerprodukt bestehend aus einer Aerosolpackungseinheit und einer Gesamtzusammensetzung, wobei die Gesamtzusammensetzung eine Haarschaumfestigerzusammensetzung nach Anspruch 1 oder 6 und ein Treibmittel ist.

17. Haar-Schaumfestigerprodukt nach Anspruch 16, **dadurch gekennzeichnet, dass** das Treibmittel ein oder mehrere Treibgase ausgewählt aus der Gruppe Kohlenwasserstoffgasen, fluorierten Gasen, Fluorkohlenwasserstoffgase, Dimethylether, Stickstoff, Luft oder Kohlendioxid oder Mischungen davon, umfasst.

18. Haarschaumfestigerprodukt nach Anspruch 17, **dadurch gekennzeichnet, dass** das Treibmittel eine Mischung aus den Treibgasen Isobutan, Propan und n-Butan ist.

19. Haarschaumfestigerprodukt nach wenigstens einem der Ansprüche 16 bis 18, **dadurch gekennzeichnet, dass** das Treibmittel eine Druckgasstufe ausgewählt aus dem Bereich von 2,7 bis 3,5 bar aufweist.

20. Haar-Schaumfestigerprodukt nach wenigstens einem der Ansprüche 16 bis 19, **dadurch gekennzeichnet, dass** der Gewichtsanteil an Treibmittel aus dem Bereich von 1,0 bis 15 Gew.-%, insbesondere 2,0 bis 12,0 Gew.-% gewählt wird, bezogen auf das Gesamtgewicht der Gesamtzusammensetzung.

21. Haarschaumfestigerprodukt nach wenigstens einem der Ansprüche 16 bis 20, **dadurch gekennzeichnet, dass** die Schaumfestigerzusammensetzungen in Form von verschäumbaren oder nachschäumbaren Zusammensetzungen vorliegen.
